# EUROPEAN PATENT SPECIFICATION

(11) **EP 1 427 428 B1**
(45) Date of publication and mention of the grant of the patent: **24.05.2006**
(21) Application number: 02759995.0
(22) Date of filing: 13.09.2002
(51) Int. Cl.: A61K 35/12, A61P 9/00, A61P 25/00

(54) **APOPTOSIS-MIMICKING NATURAL VESICLES AND USE THEREOF IN MEDICAL TREATMENT**
APOPTOSE-IMITIERENDE NATÜRLICHE VESIKEL UND DEREN VERWENDUNG ZUR MEDIZINISCHEN BEHANDLUNG
VESICULES NATURELLES SIMULANT L'APOPTOSE ET UTILISATION ASSOCIEE DANS UN TRAITEMENT MEDICAL

(30) Priority: 18.09.2001 US 323818 P
(43) Date of publication of application: 16.06.2004
(73) Proprietor: Vasogen Ireland Limited, Shannon, County Clare (IE)
(72) Inventor: BOLTON, Anthony, E., Bakeswell, Derbyshire DE45 1BJ (GB); MANDEL, Arkady, North York, Ontario M2R 3S7 (CA)
(74) Representative: Brown, David Leslie
(86) International application number: PCT/CA2002/001397
(87) International publication number: WO 2003/024419

(56) References cited:
- WO-A-99/58645
- FADOK V A ET AL: "MACROPHAGES THAT HAVE INGESTED APOPTOTIC CELLS IN VITRO INHIBIT PROINFLAMMATORY CYTOKINE PRODUCTION THROUGH AUTOCRINE/PARACRINE MECHANISMS INVOLVING TGF-BETA, PGE2, AND PAF" JOURNAL OF CLINICAL INVESTIGATION, NEW YORK, NY, US, vol. 101, no. 4, February 1998 (1998-02), pages 890-898, XP001120047 ISSN: 0021-9738
- FADOK V A ET AL: "A RECEPTOR FOR PHOSPHATIDYLSERINE-SPECIFIC CLEARANCE OF APOPTOTIC CELLS" NATURE, MACMILLAN JOURNALS LTD. LONDON, GB, vol. 405, no. 6782, 4 May 2000 (2000-05-04), pages 85-90, XP001120051 ISSN: 0028-0836
- MONASTRA G ET AL: "PHOSPHATIDYLSERINE, A PUTATIVE INHIBITOR OF TUMOR NECROSIS FACTOR, PREVENTS AUTOIMMUNE DEMYELINATION" NEUROLOGY, LIPPINCOTT WILLIAMS & WILKINS, PHILADELPHIA, US, vol. 43, no. 1, January 1993 (1993-01), pages 153-163, XP001037018 ISSN: 0028-3878

## Description

### FIELD OF THE INVENTION

This invention relates to the use of natural biological vesicles presenting phosphatidylserine on their outer membrane surface.

### BACKGROUND OF THE INVENTION

Two mechanisms of cell death in the body are recognized, necrosis and apoptosis. Apoptosis is the process of programmed cell death, described by Kerr et al in 1992 [Kerr, J.F.R., Wyllie A. H., Currie, A. R. (1992)], "Apoptosis: a basic biological phenomenon with wide-ranging implications in tissue kinetics." "British Journal of Cancer 26: 239-257," by which steady-state levels of the various organ systems and tissues in the body are maintained as continuous cell division is balanced by cell death. Cells undergoing apoptosis often exhibit distinctive morphological changes such as pronounced decrease in cell volume, modification of the cytoskeletons resulting in pronounced membrane blebbing, a condensation of the chromatin, and degradation of the DNA into oligonucleosomal fragments. Following these morphological changes, an apoptotic cell may break up into a number of small fragments known as apoptotic bodies, consisting essentiaily_of membrane-bound bodies containing intact organelles, chromatin etc. Apoptotic cells and apoptotic bodies are normally rapidly removed from the body by phagocytosis principally by macrophages and dendritic cells, before they can become lysed and release their potentially pro-inflammatory intracellular contents.

Macrophages which have ingested apoptotic cells and/or apoptotic bodies appear to inhibit pro-inflammatory cytokine production (Fadok et al., 1998) and thus may down-regulate a Th-1 response in a patient's immune system following injection of apoptotic cells or bodies, or following injection of cells susceptible to accelerated apoptosis, upon phagocytosis thereof.

During apoptosis, phosphatidylserine becomes exposed extemally on the cell membrane [Fadok V.A., Voelker D.R., Campbell P. A., Cohen, J. J., Bratton, D. L., Henson, P. M. (1992), "Exposure of phosphatidylserine on the surface of apoptotic lymphocytes triggers specific recognition and removal by macrophages." Journal of Immunology 148:2207-2216] and this exposed phosphatidylserine binds to specific receptors to mediate the uptake and dearance of apoptotic cells in mammals [Fadok V. A., Bratton, D. L., Rose, D. M., Pearson, A., Exekewitz R.A.B., Henson, P. M. (2000), "A receptor for phosphatidylserine-specific clearance of apoptotic cells," Nature 405:85-90]. The surface expression of phosphatidylserine on cells is a recognized method of identification of apoptotic cells.

International patent application PCT/EP99/03136, publication no. WO99/58645, inventors Gregoire, M. and Barthaleyns, J., discloses apoptotic bodies derived from human tumor cells recovered from a patient's tumor biopsy and induced to apoptosis, which provide effective antigens recognized by the immune system. They are reported to offer opportunitie in anti-cancer immunotherapy to enhance the specific cellular and numoral responses against tumors.

### SUMMARY OF THE INVENTION

In accordance with the present invention, natural blologl vesicles with membranes presenting phosphatidylserine on their outer membrane surface are used in manufacture of a medicament for alleviating the symptoms of (i) inflammatory diseases, as well as (ii) T-cell mediated disorders, endothelial dysfunction disorders or inappropriate cytokine expression disorders in which pro-inflammatory or anti-inflammatory cytokines are implicated. Such vesicles, upon administration to a mammalian patient, will mimic the apoptosis process with consequent down-regulation of pro-inflammatory cytokines and/or upregulation of anti-inflammatory cytokines. Immune cells can engulf the entities in an *in vivo* process resembling apoptosis, with consequent down-regulation of pro-inflammatory cytokines and/or upregulation of anti-inflammatory cytokines. Consequently, these natural biological vesicles can be used for therapeutic purposes, for treatment or prophylaxis of a wide range of mammalian disorders in which pro-inflammatory or anti-inflammatory cytokines are implicated.

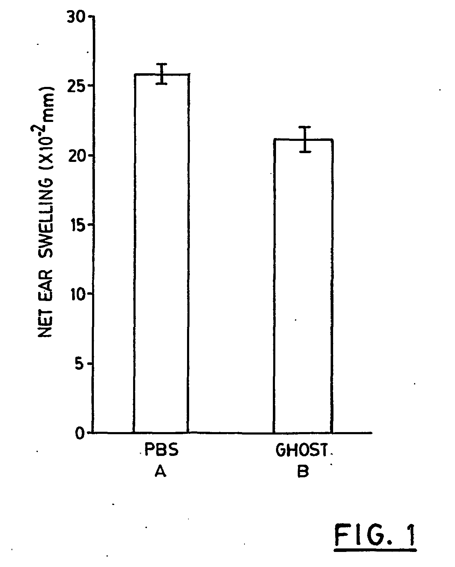

### BRIEF REFERENCE TO THE DRAWING

The accompanying FIGURE of drawings is a graphical presentation of the results of the specific experimental Example below, namely a plot of net ear swelling in a mouse model due to inflammation (contact hypersensitivity) in animals treated according to the invention and control animals.

### DESCRIPTION OF THE PREFERRED EMBODIMENTS

The natural vesicles with membranes used in the present invention are vesicles presenting phosphatidylserine (PS) on their outer membrane surfaces. On suitable introduction to the mammalian body, e.g. by intermuscular injection, antigen-presenting cells such as macrophages and dendritic cells appear to seek out the injected vesicles, and the PS groups on the membranes thereof interact with the PS receptors on the antigen-presenting cells, resulting in an apoptotic like procedure of engulfment of the vesicles with consequent down-regulation of inflammatory cytokines and/or up-regulation of anti-inflammatory cytokines.

Specific preferred natural biological vesicles presenting PS on an external membrane surface, of particular interest in the present invention, include the following:
Exosomes, which are microvesicles exfoliated from cultured cells, and may also be produced *in vivo,* e.g., during maturation of reticulocytes (see Trams et.al, *Biochimica et Physica Acta,* (1981) 645:63-70; and also Johnstone, *Biochem. Cell. Biol.,* (1982) 70:179-190);
Prostasomes, which are vesicular extracellular organelles found in seminal plasma (see Rooney et al., *J*. *Exp. Med.*, (May 1993) 177:1409-1420);
Spontaneous or induced shed membrane vesicles, i.e. membrane vesicles shed from cells as a result of inducement using detergents such as lysophasphatidylcholine, or spontaneously (see Ferber et al., *Biochimica et Biophysica Acta,* (1980) 595:244-256; also Emerson et al., *The Journal of Immunology*, (August 1981) 127(2):482-486);
Procoagulant bound to plasma membrane vesicles, i.e. thromboplastin-like activity associated with membrane vesicles, found for example in bronchoalveolar lavage fluid and derived from alveolar macrophages (see Lyberg et al., *Eur*. *Respir*. *J*., (1990) 3:61-67);
Erythrocytes with lost phospholipid asymmetry, i.e. erythrocytes with randomized, symmetric transbilayer distribution of phospholipids; these can be produced, for example, by elevating intracellular Ca⁺⁺ levels (see Pradham et al. *Molecular Membrane Biology* (1994) 11:181-188);
Activated platelets, platelets with pro-coagulant activity, which are associated with re-orientation of PS from the inner to the outer leaflet of the platelet membrane bilayer (see Bevers et.al *Biochimica et Biophysica Acta* (1983) 736:57-66); and
Platelet derived microparticles, which are membranous vesicles or microparticles shed from platelet membranes following platelet activation (see Gilbert et al., *The Journal of Biological Chemistry,* (Sept. 16, 1991) 266(26):17261-17268).

The most preferred such vesicles for use in the present invention are inside out red blood cell ghosts, which express PS on the outer surface, and sickle cell red blood cells which express PS on the surface as part of the pathology (see Schroit et al., *Biol. Cell* (1984) 51:227-238);

Natural biological vesicles presenting PS on an external membrane surface which have the property of mimicking apoptotic cells and/or apoptotic bodies in that they are phagocytosed by leukocytes of the patient's immune system with accompanying beneficial effects such as inhibition of the release of pro-inflammatory cytokines and/or promotion of the release of anti-inflammatory cytokines are provided, and are administered to patients. These natural vesicles are three dimensional bodies having shapes and dimensions ranging from those resembling mammalian cells to shapes and dimensions approximating apoptotic bodies produced by apoptosis of mammalian cells, and have PS groups on the external membrane surfaces thereof.

As noted above, exposed PS on the external membrane of a cell is known to play a key role in the clearance of apoptotic lymphocytes by macrophages. A receptor for PS is present on macrophages. A "phosphatidylserine receptor" or "PS receptor" is a receptor on an antigen presenting cell (APC), such as a macrophage, whose activity is blocked by soluble phosphatidylserine, either monomeric or oligomeric. It is contemplated that the PS receptor may also be present on other APCs, such as dendritic cells and B cells.

In a particularly preferred embodiment, the natural biological vesicles presenting PS on an external membrane surface are red cell ghosts. Erythrocytes (red blood cells) in their natural state have PS on the inner surfaces of cell membrane. When they are emptied of hemoglobin and other cellular contents, they are known as "red cell ghosts" and effectively comprise an empty vesicle of an erythrocyte membrane. These ghosts can be turned inside out, by physicavchemical means such as subjection to oxidative stress, subjection to sound energy and the like, by processes known in the art, so as to present PS on the outer surface of the membrane while maintaining vesicular form and membrane integrity. Processes for preparing inside out erythrocyte ghosts are described by Steck, T.L., 1974 "Preparation of impermeable inside-out and right-side out vesicles from erythrocyte membranes", in: *Method in Membrane Biology,* Korn, E.D. ed., Plenum Press, New York 2, 245 - 281.

There are two general types of erythrocyte ghosts, namely white ghosts, in which the hemoglobin is removed from the red blood cell without significant rupture of the membrane, and resealed ghosts, in which the membrane is opened, the contents of the cell extracted, and then the membrane is re-assembled and sealed. Either one of the two types, or a mixture of the two, can be used in the process of the present invention. The preparation of white ghosts is described by Schow, G., and Passow, H., ***Molecular and Cellular Biochemistry*** Vol.2, no. 2, 15 December 1973, pp 197 - 217. The preparation of resealed ghosts is described by Bodemann, H. and Passow, H., **J. *Membrane. Biol.,*** 8, 1 - 26 (1972) and by Rohling, O., and Neidhart, **B.**, ***Anal. Chem.*** 1999, *71*, 1077 -1082. The origin of the red cell ghosts for the present invention should be the patient himself or herself, or a compatible donor. Compatible red cells from cultured cell lines may also be used.

The use of activated platelets also constitutes a preferred embodiment of the present invention. It is known that phospholipids such as PS in the plasma membrane of human platelets are not homogeneously distributed between both halves of the membrane bilayer. In non-activated platelets, PS is almost exclusively present in the inner leaflet of the bilayer. Activation of the platelets, e.g. by simultaneous action of thrombin and collagen causes PS to be exposed at the membrane outer surface. Such activated platelets are useful in the processes of the present invention.

The natural biological vesicles presenting PS on an external membrane surface may be administered to the patient by any suitable means which brings them into operative contact with active components of the patient's immune system. Preferably, the vesicles are constituted into a liquid suspension in a biocompatible liquid such as physiological saline and administered to the patient intra-arterially, intravenously or most preferably intramuscularly or subcutaneously.

The dosages of natural biological vesicles presenting PS on an external membrane surface to be administered will vary depending on the nature of the mammalian disorder it is intended to treat and on the identity and characteristics of the patient. It is important that the effective amount of vesicles is non-harmful to the patient. The dosages and regimens needed are well within the skill of the appropriate attending clinician. When using intra-arterial, intravenous, subcutaneous or intramuscular administration of a liquid suspension of vesicles, it is preferred to administer, for each dose, from about 0.1-50 ml of liquid, containing an amount of natural PS-carrying membrane vesicles according to the invention generally equivalent to 1% - 1000% of the number of cells normally found in an equivalent volume of whole blood or the number of apoptotic bodies that can be generated from them. Generally, the number of such bodies per injection is in the range from about 500 to about 20,000,000. The number can, however, be anywhere from 500 to about 2 x 10⁹, and preferably from 10,000 to about 2 x 10⁹.

While it is not intended that the scope of the present invention should be limited by any particular theories of its mode of operation, the following is offered as a tentative explanation, for a better understanding of the ways an means by which the invention may be put into practice. It is postulated that antigen-presenting cells of the patient's immune system, notably professional antigen presenting cells (APCs), including macrophages and dendritic cells, take up the natural biological vesicles presenting PS on an external membrane surface, in a similar manner to the way in which they would take up apoptotic cells and apoptotic bodies. Having taken up the vesicles, the APCs induce an anti-inflammatory response promoting a change in the Th cell population with an increase in the proportion of Th2 cells and/or other regulatory/anti-inflammatory cell populations (e.g., Tr1 cells), and a decrease in Th1 cells. Th2 cells and other regulatory cells secrete anti-inflammatory cytokines such as interleukin-10, leading to reduced inflammation.

The present invention is indicated for use in prophylaxis and treatment of a wide variety of mammalian disorders where T-cell function, inflammation, endothelial dysfunction and inappropriate cytokine expression are involved. A patient having, suspected of having, or being particularly prone to contracting such a disorder may be selected for treatment. "Treatment" means a reduction in symptoms such as, but not limited to, a decrease in the severity or number of symptoms of the particular disorder or to limit further progression of symptoms of the disorder.

In respect of T-cell function (T-cell mediated) disorders, these are autoimmune disorders including diabetes, scleroderma, psoriasis and rheumatoid arthritis. The invention is indicated for use with inflammatory allergic reactions, organ and cell transplantation reaction disorders, and microbial infections giving rise to inflammatory reactions. It is also indicated for use in preconditioning against ingestion of poisons, exposure to toxic chemicals, radiation damage, and exposure to airborne and water-borne irritant substances, etc., which cause damaging inflammation. It is also indicated for inflammatory, allergic and T-cell-mediated disorders of internal organs such as kidney, lever, heart, etc.

With respect to disorders involving inappropriate cytokine expression for which the present invention is indicated, these include neurodegenerative diseases. Neurodegenerative diseases, including Down's syndrome, Alzheimer's disease and Parkinson's disease, are associated with increased levels of certain cytokines, including interleukin-1*β* (IL-1*β*) [see Griffin WST, Stanley, L. C., Ling, C., White, L., Macleod, V. Perrot L. HJ., White, C. L., Araoz, C., )1989). Brain interleukin 1 and S-100 immunoreactivity are elevated in Down's syndrome and Alzheimer's disease (Proceedings of the National Academy of Sciences USA 86: 7611-7615; Mogi M., Harada, M., Narabayashi, H., Inagaki, H., Minami, M., Nagatsu T. (1996)). Interleukin (IL)-1 beta, IL-1, IL-4, IL-6 and transforming growth factor-alpha levels are elevated in ventricular cerebrospinal fluid in juvenile parkinsonism and Parkinson's disease (Neuroscience Letters **211**: 13-16). It has also been shown that II-1*β* inhibits long-term potentiation in the hippocampus [Murray, C. A., Lynch, M. A. (1998). Evidence that increase hippocampal expression of the cytokine interleukin-1*β* is a common trigger for age and tress-induced impairments in long-term potentiation. Journal of Neuroscience **18**:2974-2981]. Long-term potentiation in the hippocampus is a form of synaptic plasticity and is generally considered to be an appropriate model for memory and learning [Bliss, T.V.P., Collinridge, G. L., (1993). A synaptic model of memory: long-term potentiation in the hippocampus, Nature **361**:31-39]. Thus, inappropriate cytokine expression in the brain is currently believed to be involved in the development and progression of neurodegenerative diseases.

Thus, the invention is indicated for the treatment and prophylaxis of a wide variety of mammalian neurodegenerative and other neurological disorders, including Downs syndrome, Alzheimer's disease, Parkinson's disease, senile dementia, depression, multiple sclerosis, Huntington's disease, peripheral neuropathies, spinal cord diseases, neuropathic joint diseases, chronic inflammatory demyelinating disease, neuropathies including mononeuropathy, polyneuropathy, symmetrical distal sensory neuropathy, neuromuscular junction disorders, myasthenias and amyotrophic lateral sclerosis.

Regarding disorders involving endothelial dysfunction, the present invention is indicated for the treatment and prophylaxis of a wide variety of such mammalian disorders including, but not limited to, cardiovascular diseases, such as atherosclerosis, peripheral vascular disease, congestive heart failure, stroke, myocardial infarction, angina, hypertension, etc., vasospastic disorders such as Raynaud's disease, cardiac syndrome X, migraine etc., and the damage resulting from ischemia (ischemic injury or ischemia-reperfusion injury). In summary, it can be substantially any disorder that results from an inappropriately functioning endothelium.

The invention is further described for illustrative purposes in the following specific example.

### EXAMPLE

This example shows the effect of injecting inside out red blood cell ghosts on ear swelling in the murine contact hypersensitivity(CHS) model. This is a well known and well accepted preclinical model of inflammation consequent upon Th-1 cell downregulation or switch to Th-2 phenotype.

The ghosts were prepared from sodium citrated blood obtained from syngeneic mice. The blood was diluted with an equal volume of ice-cold isotonic phosphate buffered saline (PBS) (300mOsm). After centrifugation at 2000 rpm for 5 minutes at room temperature, the plasma and buffy coat were removed and washed again 3x with cold isotonic PBS. A 50% cell suspension (v/v) was made up in the phosphate buffer. Hemolysis was performed by adding 1:15 phosphate buffer (20mOsm). This solution was left to sit on ice for 10 minutes. Ghosts were resealed by incubating the cells at 37 degrees C for 40 minutes. Cells were then centrifuged for 5 minutes at 2000 rpm at room temperature and re-suspended in isotonic PBS.

Female BALB/c mice, age 6-8 weeks, weighing 22-25 g wre obtained from Jackson Laboratories. Red blood cell ghosts prepared as above

Some mice were assigned to group A, control, and received no injections. Other mice were assigned to group B, and received injections of suspensions of red cell ghosts.

The experiments were carried out over 7 days. Sensitization took place on day 1. For sensitization purposes, mice of group B received their red cell ghost injections for day 1, and were anesthetized using 0.2 ml intraperitoneal (IP) injection of 5 mg/ml pentobarbital sodium. The abdominal skin of the mouse was sprayed with 70% ETOH. A blade was used to remove about a one-inch diameter of hair from the abdomen. The bare area was painted with 25 µl of 0.5% 2,4-dinitrofluorobenzene (DNFB) in 4:1 acetone:olive oil using a pipette tip. Control mice of group A were similarly sensitized, on the same day.

On each of days 1, 2, 3, 4, 5, 6 and 7, experimental mice were injected with the red cell ghosts suspended in physiological saline, 50 *µ*l volume containing about 600,000 bodies, via intramuscular (IM) injection. On Day 6, following injection for that day, mice were challenged with DNFB as follows: 10 *µ*l of 0.2%DNFB was painted on the dorsal surface of the right ear with a pipette tip and 10 *µ*l of vehicle was painted on the left ear with a pipette tip.

On Day 7, 24 hours after challenge, ear thickness was measured using a Peacock spring loaded micrometer, the animals being locally anesthetized with Halothane. Increase in ear swelling was used as a measure of CHS response. Data is expressed as the difference in the treated right ear thickness minus the thickness of the vehicle treated left ear, in microns. The results are shown in the accompanying Figure, a bar graph showing net ear swelling (mean of the animals in each group). Bar A is results from the control group, bar B those from the animals treated with the ghost compositions. The significance of difference between the two experimental groups was determined by the two-tailed student t test. A value of p<0.05 was considered significant. Experimental group B shows statistically significant improvement (18%) over control group, which received no injections.

## Claims

1. Use in manufacture of a medicament for alleviating the symptoms of (i) inflammatory diseases, as well as (ii) T-cell mediated disorders, endothelial dysfunction disorders or inappropriate cytokine expression disorders, in which pro-inflammatory or anti-inflammatory cytokines are implicated, of natural biological vesicles presenting phosphatidylserine on their outer membrane surface.

2. Use according to claim 1 wherein the natural biological vesicles are selected from exosomes; prostasomes; spontaneous or induced shed membrane vesicles; procoagulant bound to plasma membrane vesicles; inside out red blood cell ghosts; sickle cell red blood cells; erythrocytes with lost phospholipid asymmetry; activated platelets; platelets with procoagulant activity; and platelets derived microparticles.

3. Use according to claim 1 or claim 2, wherein the vesicles are inside out red blood cell ghosts.

4. Use according to any preceding claim, wherein the vesicles are used in quantities of from 500 - 10⁹ per-unit dosage.

5. Use according to any preceding claim, in the treatment or prophylaxis of an autoimmune disease in the patient.

6. Use according to any of claims 1-4, in the treatment or prophylaxis of a neurodegenerative disease in the patient.

7. Use according to any of claims 1-4, in the treatment or prophylaxis of a defective endothelial dysfunction disorder in the patient.

8. Use according to claim 3, wherein the red blood cell ghosts are used in quantities of from 500 - 10⁹ per-unit dosage.

9. Use according to any preceding claim, for administration by intramuscular injection.

10. Use according to any one of claims 1, 2, 4 to 7 or 9, wherein the natural biological vesicles are activated platelets.

11. Use according to any one of claims 1, 2, 4 to 7 or 9, wherein the natural biological vesicles are sickle cell red blood cells.

## Patentansprüche

1. Verwendung von natürlichen, biologischen, auf der Oberfläche ihrer äußeren Membran Phosphatidylserin präsentierenden Vesikeln zur Herstellung eines Arzneimittels zur Linderung der Symptome von (i) entzündlichen Erkrankungen sowie (ii) T-Zell-vermittelten Störungen, Störungen durch endotheliale Dysfunktion oder Störungen durch fehlangepasste Cytokin-Expression, bei denen entzündungsfördernde oder antientzündliche Cytokine beteiligt sind.

2. Verwendung nach Anspruch 1, worin die natürlichen biologischen Vesikel aus Exosomen, Prostasomen, spontan oder induziert abgesonderten Membranvesikeln, mit über eine Gerinnungsvorstufe an Plasma gebundenen Membranvesikeln, Erythrozytenghosts mit nach außen gestülpter Innenseite, Sichelzellerythrozyten, Erythrozyten mit Verlust der Phospholipidasymmetrie, aktivierten Thrombozyten, Thrombozyten mit präaktivierter Coagulationsaktivität und von Thrombozyten stammenden Mikropartikeln ausgewählt sind.

3. Verwendung nach Anspruch 1 und 2, worin die Vesikel Erythrozytenghosts mit nach außen gestülpter Innenseite sind.

4. Verwendung nach einem der vorhergehenden Ansprüche, worin die Vesikel in Mengen von 500 bis 10⁹ pro Einheitsdosis verwendet werden.

5. Verwendung nach einem der vorhergehenden Ansprüche bei der Behandlung oder Prophylaxe einer Autoimmunerkrankung bei einem Patienten.

6. Verwendung nach einem der Ansprüche 1 bis 4 bei der Behandlung oder Prophylaxe einer neurodegenerativen Erkrankung bei einem Patienten.

7. Verwendung nach einem der Ansprüche 1 bis 4 bei der Behandlung oder Prophylaxe einer durch die Dysfunktion eines geschädigten Endothels hervorgerufenen Störung bei einem Patienten.

8. Verwendung nach Anspruch 3, worin die Erythrozytenghosts in Mengen von 500 bis 10⁹ pro Einheitsdosis verwendet werden.

9. Verwendung nach einem der vorhergehenden Ansprüche zur Verabreichung durch intramuskuläre Injektion.

10. Verwendung nach einem der Ansprüche 1, 2, 4 bis 7 oder 9, worin die natürlichen biologischen Vesikel aktivierte Thrombozyten sind.

11. Verwendung nach einem der Ansprüche 1, 2, 4 bis 7 oder 9, worin die natürlichen biologischen Vesikel Sichelzellerythrozyten sind.

12. Verwendung nach einem der Ansprüche 1, 2, 4 bis 7 oder 9, worin die natürlichen biologischen Vesikel Exosome sind.

## Revendications

1. Utilisation dans la fabrication d'un médicament pour éviter les symptômes de (i) maladies inflammatoires, ainsi que de (ii) troubles induits par des cellules T, dysfonctionnements endothéliaux ou dérégulations inadéquates dans l'expression de la cytokine, dans lesquelles les cytokines anti-inflammatoires ou pro-inflammatoires sont impliquées, de vésicules biologiques naturelles présentant de la phosphatidylsérine sur la surface de leur membrane externe.

2. Utilisation selon la revendication 1, dans laquelle les vésicules biologiques naturelles sont sélectionnées parmi les exosomes ; les prostasomes ; les vésicules dépouillées de leurs membranes de manière induite ou spontanée; les vésicules ayant un procoagulant lié à la membrane plasmique; les fantômes de globules rouges inversés ; les drépanocytes ; les érythrocytes avec une asymétrie phospholipidique perdue ; les plaquettes activées ; les plaquettes ayant une activité procoagulante ; et les microparticules dérivées des plaquettes.

3. Utilisation selon la revendication 1 ou 2, dans laquelle les vésicules sont des fantômes de globules rouges inversés.

4. Utilisation selon l'une quelconque des revendications précédentes, dans laquelle les vésicules sont utilisées en des quantités allant de 500 à 10⁹ par unité de posologie.

5. Utilisation selon l'une quelconque des revendications précédentes dans le traitement ou la prophylaxie d'une maladie autoimmune chez le patient.

6. Utilisation selon l'une quelconque des revendications 1 à 4 dans le traitement ou la prophylaxie d'une maladie neurodégénérative chez le patient.

7. Utilisation selon l'une quelconque des revendications 1 à 4 dans le traitement ou la prophylaxie d'un dysfonctionnement endothélial défectif chez le patient.

8. Utilisation selon la revendication 3, dans laquelle les fantômes de globules rouges sont utilisés en des quantités allant de 500 à 10⁹ par unité de posologie.

9. Utilisation selon l'une quelconque des revendications précédentes pour l'administration par injection intramusculaire.

10. Utilisation selon l'une quelconque des revendications 1, 2, 4 à 7 ou 9, dans laquelle les vésicules biologiques naturelles sont des plaquettes activées.

11. Utilisation selon l'une quelconque des revendications 1, 2, 4 à 7 ou 9, dans laquelle les vésicules biologiques naturelles sont des drépanocytes.

12. Utilisation selon l'une quelconque des revendications 1, 2, 4 à 7 ou 9, dans laquelle les vésicules biologiques naturelles sont des exosomes.
